(19) Europäisches Patentamt
European Patent Office
Office européen des brevets

(11) Publication number: 0 281 395 A2

(12) # EUROPEAN PATENT APPLICATION

(21) Application number: 88301850.9

(22) Date of filing: 03.03.88

(51) Int. Cl.$^4$: A 61 K 7/48

(30) Priority: 06.03.87 US 26438

(43) Date of publication of application: 07.09.88 Bulletin 88/36

(84) Designated Contracting States: AT BE CH DE FR GB GR IT LI LU NL SE

(71) Applicant: Richardson-Vicks, Inc.
Ten Westport Road
Wilton, CT 06897 (US)

(72) Inventor: Kendall, Joyce Marie
162 Stonybrook Road
Stratford, CT 06497 (US)

Maes, Daniel H.
29 Heartstone Lane
Monroe, CT 06468 (US)

Figueroa, Ramon, Jr.
87 Christian Street
Oxford, CT 06483 (US)

(74) Representative: Brooks, Maxim Courtney et al
Procter & Gamble (NTC) Limited Whitley Road
Longbenton
Newcastle-upon-Tyne NE12 9TS (GB)

(54) **Improved skin moisturizing emulsions.**

(57) Skin care emulsion compositions containing a water-absorbing resin polymerizate in an aqueous phase for improved skin moisturization.

EP 0 281 395 A2

## Description

# IMPROVED SKIN MOISTURIZING EMULSIONS

### Background of the Invention

The present invention relates to cosmetic skin care preparations of the emulsion type having improved skin moisturizing activity by the addition of a particular gellant resin material to the aqueous phase of the emulsion.

Single emulsion skin care preparations, such as lotions and creams, of the oil-in-water (o/w) type and water-in-oil (w/o) type are well known in the cosmetic art and are in daily use by consumers, the o/w type being more commonly utilized. Also reported are water-in-oil type personal care emulsions which have a silicone component as the oil phase, for example see U.S. Patent No. 4,311,695. Multiphase (i.e., double) emulsion compositions, such as the water-in-oil-in-water (w/o/w) type, for example see U.S. Patent No 4,254,105, and the oil-in-water-in-oil (o/w/o) type, are of lesser commercial significance to date in the cosmetic field. In general, such single or multiphase emulsions contain water, emollients and emulsifiers as essential ingredients and cosmetically acceptable optional additives such as preservatives, antioxidants, skin conditioners, humectants, thickeners, cleansers, colorants, odorants and other components generally utilized in the cosmetic art.

The emollient nature of such emulsions may provide beneficial skin moisturizing and softening activities by the addition of sufficient quantities of such well known cosmetic emollients and humectants as mineral oil, petrolatum, lanolin, glycerin, propylene glycol and vegetable oils, to name but a few.

The subject invention now provides the use of a particular gellant resin material as a moisturizing component in skin care emulsion compositions.

### Detailed Description of the Invention

It has now been found that a marked increase in moisturizing activity in skin care emulsion compositions may be obtained by incorporating into the aqueous phase of the particular emulsion system a particular starch- or cellulose-based water-absorbent resin. The disclosure of U.S. Patent No. 4,076,663, incorporated herein by reference, is pertinent background for this invention because it discloses said resins and their process of preparation.

According to said patent, the process of preparing said resins "... comprises polymerizing (A) at least one polysaccharide selected from the class consisting of starches and celluloses, (B) at least one monomer having a polymerizable double bond which is water-soluble or becomes water-soluble by hydrolysis, and (C) a cross-linking agent, and subjecting, if necessary, the resulting product to hydrolysis". (Column 1, lines 40-46).

The patent disclosure identifies and exemplifies in detail the various embodiments of the starch and cellulose polysaccharide component (A), the ethylenically unsaturated monomer component (B) and the cross-linking agent component (C) that are utilizable in preparing the polymerized water-absorbing resin composition obtained therefrom, all of which embodiments and resultant polymerized resins are intended to be embraced within the scope of this invention.

As used herein, the term "water-absorbing resin polymerizate" is broadly meant to encompass the entirety of the starch-and cellulose-based water-absorbent polymerized resin compositions which may be prepared according to the process disclosed in the aforementioned U.S. Patent No. 4,076,663. The term "starch-based water-absorbing resin polymerizate" is meant to include any of said water-absorbing resin compositions wherein component (A) is a starch.

The starch-based water-absorbing resin polymerizates preferred for use in the subject invention are those obtainable by polymerization of those components (A), (B) and (C) identified as "preferred" in the 4,076,663 patent. Accordingly, the preferred component (A) polysaccharides are:

"... natural starches such as wheat starch and corn starch, and alpha-starches thereof ...". Column 1, lines 62-64);

the preferred component (B) water-soluble monomers are:

"... carboxyl group-containing monomers such as (meth)acrylic acid and maleic acid anhydride; carboxylic acid salt group-containing monomers such as sodium (meth)acrylate, trimethylamine (meth)acrylate, and quaternary ammonium salt group-containing monomers such as N,N,N-trimethyl-N-(meth)acryloyloxyethylammonium chloride". (Column 3, lines 20-28);

and the preferred component (C) cross-linking agents are:

"... a diester of (meth)acrylic acid with an alkylene ($C_2$-$C_6$) glycol, polyoxyalkylene ($C_2$-$C_4$) glycol having a molecular weight of less than 400, bis(meth)acrylamide, and oxides of alkaline earth metals and zinc". (Column 4, lines 35-39).

The most preferred starch-based water-absorbing resin polymerizates are those generically designated as starch-acrylate graft polymers and obtainable by polymerization of a natural starch as the component (A) polysaccharide; a water-soluble monoethylenically unsaturated monomer having a polymerizable double bond and selected from the group consisting of (meth)acrylic acid, anhydride thereof and salt thereof, for example, an alkali metal salt, most preferably sodium, as the component (B) water-soluble monomer; and the component (C) cross-linking agent, for example, N,N-alkylene($C_1$-$C_6$)bis-(meth)acrylamide, the N,N-methylenebisacrylamide being most preferred. When sodium (meth)acrylate is utilized as monomer (B), the resultant

polymerizate is also referred to as a "starch grated sodium polyacrylate".

A particularly suitable commercial preparation of a starch grafted sodium polyacrylate is available from the U.S. supplier, Celanese Superabsorbent Materials, Portsmith, VA, and marketed under the "SANWET" trademark in numerically identified grades such as SANWET® IM-1000, IM-1500 and IM-2500. This commercial preparation, according to said supplier, has as its main component a starch grafted sodium polyacrylate which can be structurally represented as follows:

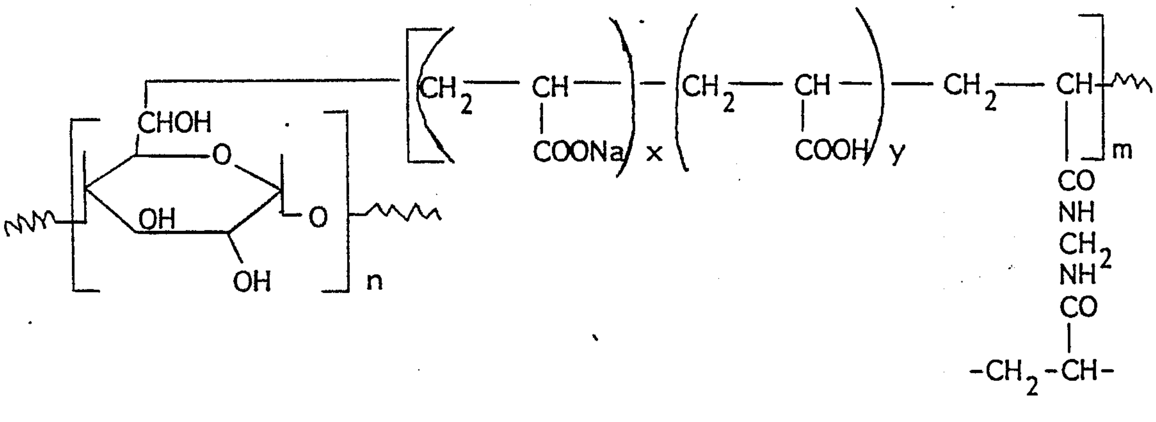

and also contains some low and high molecular weight sodium polyacrylate and some low molecular weight starch as minor components and a small amount of moisture.

According to U.S. Patent 4,076,663, the water-absorbing resin polymerizates, which are readily obtainable as end products in granular or dry powder form, are highly water-absorbent and provide excellent absorbence for water and for body fluids such as blood, urine, etc., which characteristics provide appropriate applications in absorbent dressings and products such as paper diapers, sanitary napkins, gauze, paper towels, etc. (for example, see U.S. Patent No. 4,610,678).

It has now been found that a skin moisturizing emulsion composition may be provided wherein an aqueous phase of the emulsion contains an effective skin moisturizing amount of said water-absorbing resin polymerizate. In general, at least about 0.01 weight percent and, preferably, from about 0.1 to about 5 weight percent, based on the total weight of the emulsion composition, of the water-absorbing resin polymerizate will be sufficient to effectivlye provide or enhance the moisturizing activity of the emulsion composition. More than 5 weight percent may also be utilized but is deemed unnecessary from both a benefit-to-weight ratio and the aesthetic appearance of the final composition. Preferably, the water-absorbing resin polymerizate is dispersed in the external continuous aqueous phase of an oil-in-water (most preferred) or a water-in-oil-in-water emulsion system, although it may be advantageously incorporated into the aqueous discontinuous phase of a water-in-oil or an oil-in-water-in-oil emulsion system. The water absorbing resin polymerizate may also be utilized in the aqueous phase of single or multiphase emulsion systems wherein a silicone fluid is utilized as the oil-phase component, for example, in the water-in-silicone personal-care emulsions of U.S. Patent No. 4,311,695, and in oil-in-water-silicone personal care emulsions, such as, for example, Example VII hereinafter.

Although the water-absorbing resin polymerizates are excellent gellant materials, they are essentially water insoluble and their addition to water or the water phase results in a gel or slurry of individual hydrated particles with a somewhat curdled appearance which is not deemed advantageous from a cosmetic elegance point of view. The use of mechanical high shear dispersing means, such as a Gifford-Wood Homogenizer, a suitable colloid mill, a Ross Mixer, an Arde-Barenco, an IKA SD40 Super Dispax In-Line Tekmar and the like, may be used to reduce the particle size of the hydrated gellant material to obtain a cosmetically acceptable dispersion.

It has also been found that the presence of a small amount, for example 0.5-2.0 percent by weight, of a lipid component, which can either be added to the aqueous phase before dispersion of the water-absorbing resin polymerizate, or used to wet and disperse the water-absorbing resin polymerizate therein before dispersion into the aqueous phase, will assist formation of a smoother, more aesthetic, gel. Typical such lipid components include, for example, heavy mineral oil, vegetable oils, fatty ($C_{12}$-$C_{18}$) alcohols, fatty ($C_{12}$-$C_{18}$) esters particularly ethoxylated esters such as laureth-2-acetate, laureth-2-benzoate, glycereth-7-triacetate or mixture thereof, and the like.

It has further been found that the moisturizing activity of the final emulsion composition is increased by utilizing finer particulate grades of the water-absorbing resin polymerizate, as illustrated in Examples IX and X hereinafter. It is preferred, therefore, that the water-absorbing resin polymerizates have a particle size distribution of about 10% maximum greater than 200 mesh and about 50% minimum less than 325 mesh as measured by Standard U.S. Series Sieves with cover and collector pan.

As noted previously, single and multiphase cosmetic emulsion compositions such as, for example, lotions and creams, containing one or more optional cosmetically acceptable additive ingredients, are well-known product forms and the preparation thereof is well within the skill of the cosmetic formulator. The emulsion compositions of this invention, therefore, are readily prepared by using art-recognized principles and

methodologies in mixing the ingredients together and in choosing the type of mixing or dispersing equipment to be used. The Examples hereinafter provide illustrative components, both essential and optional in cosmetic skin care emulsions and illustrate procedures for making the subject compositions.

In general, the aqueous phase of the particular emulsion composition is prepared with water and the water-soluble or water-miscible ingredients other than the water-absorbing resin polymerizate. Elevated temperatures of about 75-85° and thorough agitation are usually employed to enhance uniform solubility or dispersion of ingredients. The water-absorbing resin polymerizate in powder or granular form, or in aqueous or lipid dispersion form, is then slowly added to the heated aqueous phase with high shear dispersing means until a uniform dispersion of the water-absorbing resin polymerizate is obtained. The aqueous phase containing the polymerizate may then be used to form part of the desired single or multiphase emulsion.

In the emulsion composition of this invention, it is essential that there be at least one water phase having the water-absorbing resin polymerizate uniformly dispersed therein, at least one non-aqueous phase such as an oil or silicone phase, and an appropriate dispersing agent or emulsifier for dispersing one of said phases in the other. It will be understood that one or more cosmetically acceptable ingredients may be included in the subject emulsions to provide both a cosmetically elegant and multifunctional product, for example, one with moisturizing and sun-screening activities (a preferred embodiment of this invention), one with moisturizing and skin conditioning activities, and the like. Such ingredients include one or more of a diluent, thickener, stabilizer, humectant, emollient, preservative, anti-oxidant, colorant, odorant, conditioner, auxiliary emulsifier, opacifier, U.V.-absorbing sunscreen agent and other conventional cosmetically acceptable additives. Depending upon the solubility or miscibility characteristic of the particular additive, it can be incorporated into whichever emulsion phase is most suitable.

It has been found, and confirmed by the experiments described in the following examples, that skin care emulsion compositions having at least one aqueous phase as part of the emulsion may be improved by uniformly dispersing an effective moisturizing amount of the previously described water-absorbing resin polymerizate, preferably a starch-based water-absorbing resin polymerizate, into such aqueous phase. The emulsion compositions of this invention, when applied to the skin, will provide enhanced cosmetically desirable effects as compared to the same composition absent said water-absorbing resin polymerizate. In particular, the subject emulsion compositions provide enhanced moisturization and softening of the skin. Other beneficial effects include the smoothing of wrinkles, improvement of the mechanical properties of the skin such as elasticity and tissue tension, and an increase of the moisture retaining capacity of the skin. Existing wrinkles are smoothed and the withered, unelastic state of the skin is clearly improved. For example, in standard wrinkle reduction tests wherein the composition to be tested is applied to particular skin areas of human subjects, such as the eye or forehead area, the subject emulsion compositions containing the water-absorbing resin polymerizate will generally provide a marked reduction in wrinkle depth, length and number, commonly referred to as superficial facial lines (SFL). In comparison tests with similar compositions but absent the water-absorbing resin polymerizate, a reduction in SFL measurements of at least about 20 percent will generally be observed. A typical testing procedure for measuring SFL reduction is reported by E. W. Packman and E. H. Gans, "Topical Moisturizers: Quantification of Their Effect on Superficial Facial Lines", J. Soc. Cosmetic Chemists, Vol. 29, p. 79-90. Feb., 1980.

Skin Softness Test

The enhanced moisturizing effect obtained on human skin following topical application of the subject emulsion compositions is demonstrable in a procedure for measuring skin softness (hereinafter sometimes referred to as the "Skin Softness Test") using an instrument known as a Gas Bearing Electrodynamometer. This procedure was first described by M. Christensen et al in J. Invest. Dermatol., 69, 282-286 (1977) and then modified by D. Maes et al in Intern. J. Cosmetic Science, 5 189-200, (1983). The measurement of the effect of a moisturizer on the softness of the skin has been shown to be correlated to the extent of moisturization of the superficial layers of the skin (Stratum Corneum) by S. Jaques et al in Bioengineering and the Skin, Vol. 1. No. 3, July, 1985, p. 260.

The Gas Bearing Electrodynamometer is an instrument that accurately measures the displacement of, and the force acting on a moving armature. This force is produced by a coil moving in a uniform magnetic field, and therefore is directly proportional to the intensity of the current circulating in the coil. The coil is activated by a Low Frequency Function Generator (Hewlett Packard model 3310A) operating in a sinusoidal mode at a frequency of 0.3 cycle sec $^{-1}$ (0.3 Hz). The displacement of the moving armature is measured by a sensitive Linear Differential Transformer (Schaevitz 050 HR) which is mounted coaxially with the force coil.

Mechanical friction during the movement of the armature is eliminated by gas bearing of the moving parts; this technical feature provides the coil with a suspension of highest compliance. The attachment of the moving armature to the skin is established by a stiff wire probe having a small stub at the free end. This is securely fastened to the skin surface by a disc or double sided adhesive tape.

The force (stress) and displacement (strain) signals are recorded by a storage oscilloscope (Tektronix model 5111). The screen display has a form of hysteresis loop with force on the vertical axis and displacement on the horizontal axis.

The analysis of the hysteresis loop allows the disclosure of a number of biomechanical characteristics of the Stratum Corneum such as the softness parameter we already described but also of the viscous response of this material, resulting in a phase lag between strain and stress.

The stress and the strain signals are calibrated in grams and mm respectively, which allows the skin softness to be calculated in mm per gram. The higher the displacement per unit force, the softer the skin and vice-versa; stiff, dry skin is thus characterized by a small displacement.

In-Vivo Measurements:

The measurements of the skin softness parameter are carried out in vivo on a group of ten selected subjects with various skin conditions varying from stiff (dry) to soft skin. The measurements take place on the back of the hands which are precisely positioned by insertion of the fingers into a precast plaster mold. Such an arrangement is found to provide enough stability to the subjects' hand to avoid any interfering movement during the measurement of the skin softness parameter.

The evaluation of the effect of the test product on skin softness is done before product application (baseline) and at standardized time intervals after product application to the subjects' skin at a dose of 2 $mg/cm^2$.

The speed with which the loops are obtained on the oscilloscope screen (one loop every three seconds) allows the observation of rapid changes in the skin softness after product application. In this way, it is possible to test both the short-term effects (e.g., 2 mins. after product application) as well as the possible long-lasting effect of the product (e.g., up to six hours).

In-Vitro Measurements:

The measurement of skin softness can be carried out in vitro on pieces of excised full thickness human skin. Pieces of human skin (2" x 3/4") devoid of any adipous tissue are glued on a glass plate in such a manner that the sides of the specimen are sealed by the glue in order to avoid premature drying of the sample. The skin slabs are conditioned for twelve hours before the measurement in a room which is maintained at a standardized temperature (20°C) and humidity (25% RH). The application of the test product and the measurement of skin softness are performed in a similar manner to the in vivo experiment. The effect of each test product on the skin softness is measured on three separate pieces of skin.

Calculations:

The percent increase in skin softness measured after application of each composition is calculated using the following formula:

$$\text{Percent increase at time t.} = \frac{\text{Softness (t)} - \text{Softness (o)}}{\text{Softness (o)}} \times 100$$

wherein "Softness (t)" is the value of skin softness measured in um/g at the time after treatment; and "Softness (o)" is the value of skin softness measured before treatment in um/g. The reproducibility of the measurements of skin softness, when carried out in vivo on the back of the hand of a single subject, results in a standard deviation which is equal to 3.2% of the mean value.

Softness Index (S.I.):

The Softness Index represents the total improvement in softness measured after treatment with a test product as compared to a control. This index is obtained by plotting a curve of percent increase at time t of the respective test product and control. The area under each curve is then measured.

$$\text{Softness Index (S.I.)} - \frac{\text{Area under curve (test product)}}{\text{Area under curve (control)}}$$

The higher the S.I. value, the greater the skin softening effect.

The following examples are intended to illustrate, without limitation thereto, the subject invention.

Example 1

An oil-in-water sunscreen emulsion with an anionic emulsifier system is made from the following ingredients indicated by their chemical or Cosmetic, Toiletry and Fragrance Association (CTFA) name in the form of a light cream. The emulsion, which does not contain a water-absorbing resin polymerizate in its aqueous phase, demonstrates a cosmetic skin care emulsion base to which said resin polymerizate may be incorporated, as show in subsequent examples.

| Ingredient | % w/w |
|---|---|
| Water Phase: | |
| Methylparaben (preservative) | 0.20 |
| Pantethine (moisturizer) | 0.10 |
| Carbomer 934 (thickener) | 0.08 |
| Sodium hydroxide, 10% (neutralizer) | 1.00 |
| Purified water, q.s. to 100% w/w | |
| Oil Phase: | |
| Heavy mineral oil | 4.00 |
| Stearic acid, double pressed (anionic emulsifier) | 3.00 |
| Cholesterol (auxiliary emulsifier) | 1.00 |
| Cetyl alcohol (auxiliary emulsifier) | 1.80 |
| Castor oil (emollient) | 1.00 |
| Cetyl palmitate (emollient) | 1.20 |
| Octyl dimethyl PABA (U.V.-absorber) | 1.40 |
| Propylparaben (preservative) | 0.10 |

In a mixing vessel equipped with a mechanical stirrer, water and the water phase ingredients other than the sodium hydroxide aqueous solution are added and mixed with heating to about 75-80°C to form a uniform aqueous dispersion. The sodium hydroxide solution is then added and mixed into the aqueous phase to neutralize the acidic Carbomer thickener.

In a separate mixing vessel, the mineral oil and oil phase ingredients are added and mixed with heating to about 80-82°C to form a uniform oil phase. The heated oil phase is slowly added to the heated water phase using high speed mechanical dispersing means and mixing is continued until a homogeneous o/w emulsion is obtained and through cooling to room temperature. If desired, optional colorants such as water-soluble dyes are preferably mixed into the emulsion at about 45-50°C and fragrant oils are preferably added at about 35-40°C.

Example 11

The oil-in-water emusion of Example 1 is modified by uniformly dispersing 0.01 weight percent of a starch-based water-absorbing resin polymerizate into the aqueous phase. Celanese's starch grafted sodium polyacrylate product, SANWET IM-1000, powder, find grind (FG), is employed as the gellant resin material. After the sodium hydroxide neutralization step of Example I, the SANWET IM-1000 is slowly added to the heated aqueous phase with vigorous shearing in a vessel equipped with a high speed dispersing means until a homogeneous dispersion is obtained with an average particle size of less than 50 microns (Hegman 250-0 micron gauge). The oil phase is prepared and added as in Example 1 to complete the o/w emulsion.

The thus-obtained o/w emulsion with the gellant resin material is tested for moisturizing activity in a direct comparison test with the o/w emulsion of Example 1 without said gellant. The following results, obtained from the Skin Softness Test hereinbefore described, illustrate the superior moisturizing activity of the o/w emulsion of this example.

% Increase in Skin Softness (in vivo)

| Time | o/w Emulsion of Ex. I | o/w Emulsion of Ex. I with 0.01% w/w SANWET IM-1000 |
|---|---|---|
| 0 (baseline) | 0 | 0 |
| 2 mins. | 37.9% | 42.7% |
| 5 " | 30.3 | 41.3 |
| 10 " | 20.4 | 37.4 |
| 1 hour(s) | 12.4 | 24.9 |
| 2 " | 7.2 | 18.5 |
| 3 " | 6.1 | 18.0 |
| 4 " | 5.9 | 18.0 |
| 5 " | 5.7 | 16.1 |
| 6 " | 6.2 | 17.7 |
| S.I. | 1.0 | 2.33 |

Example III

The oil-in-water emulsion of Example I is modified by incorporating 0.1 weight percent of SANWET IM-1000 (FG) into the aqueous phase by following the procedure of Example II and the resultant o/w emulsion is compared for moisturizing activity against the base emulsion of Example I. The tabulated data illustrate the improved moisturizing effect attributable to the starch-based water-absorbing resin polymerizate.

% Increase in Skin Softness (in vivo)

| Time | o/w Emulsion of Ex. I | o/w Emulsion of Ex. I with 0.1% w/w SANWET IM-1000 |
|---|---|---|
| 0 (baseline) | 0 | 0 |
| 2 mins. | 33.8% | 43.4% |
| 5 " | 22.8 | 33.4 |
| 10 " | 22.0 | 34.6 |
| 1 hour(s) | 14.7 | 24.5 |
| 2 " | 12.0 | 17.0 |
| 3 " | 11.4 | 14.8 |

| Time | % Increase in Skin Softness (in vivo) o/w Emulsion of Ex. I | o/w Emulsion of Ex. I with 0.1% w/w SANWET IM-1000 |
|---|---|---|
| 4 hours | 7.6 | 24.1 |
| 5 " | 6.4 | 15.9 |
| 6 " | 5.7 | 10.4 |
| S.I. | 1.0 | 2.1 |

The creamy emulsion composition of this example is rubbed on the skin in the conventional manner for applying cosmetic skin lotions, leaving the skin with a non-oily coating that, in addition to providing sun-protective activity, moisturizes and softens the skin.

Example IV

In this example, an oil-in-water sunscreen emulsion lotion with a nonionic emulsifier system is formed from the following ingredients (indicated by chemical or CTFA name) by the general procedure of Example 1. The emulsion, which does not contain a water-absorbing resin polymerizate is utilized as a base formulation (control) in the next example.

| Ingredient | % w/w |
|---|---|
| Water Phase: | |
| Methylparaben (preservative) | 0.225 |
| Pantethine (moisturizer) | 0.1 |
| Imidazolidinyl urea (preservative) | 0.2 |
| Colloidal oatmeal (skin conditioner) | 0.4 |
| Glycereth-7-triacetate (emollient)* | 1.0 |
| Purified water, q.s. to 100% w/w | |
| Oil Phase: | |
| Heavy mineral oil | 2.0 |
| Cholesterol (auxiliary emulsifier) | 1.2 |
| Octyl methoxycinnamate (U.V. absorber) | 2.0 |
| PPG-20 Methyl glucose ether distearate (emollient & auxiliary emulsifier) | 0.4 |

| Ingredient | % w/w |
|---|---|
| Oil Phase: (Continued) | |
| Propylparaben (preservative) | 0.15 |
| Hydrogenated palm glycerides and cetyl alcohol and TEA-isostearoyl hydrolyzed collagen (primary nonionic emsulsifier)** | 1.6 |
| Cetyl alcohol (auxiliary emulsifier) | 0.2 |
| Myristyl alcohol (auxiliary emulsifier) | 0.2 |
| Cyclomethicone (lubricant) | 0.5 |

* Commercially available from Phoenix Chemicals, Inc., Somerville, N.J., under the trade name "Pelemol G7A".

** Commercially available from Inolex Chemical Co., Chicago, IL, under the trade name LEXEMUL® LPM".

Example V

The oil-in-water emulsion of Example IV is modified by incorporating 0.4 weight percent of SANWET IM-1000 (FG) into the aqueous phase. In a mixing vessel equipped with a mechanical stirrer, the water and water phase ingredients, other than the glycereth-7-triacetate, are added and mixed with heating to about 75-82°C to form a uniform aqueous dispersion. This phase is then transferred to a vessel equipped with an IKA SD40 Super Dispax In-Line Tekmar with a mixer affixed to the top of the vessel.

In a separate container the SANWET IM-1000 (0.4%) is added to the glycereth-7-triacetate to form a wetted dispersion of the resin gellant. This dispersion is slowly added into the vortex formed by the mixing of the aqueous phase with recirculation through the In-Line Tekmar until a uniform dispersion of the resin gellant is obtained with an average particle size of 50 microns on a Hegman gauge. The aqueous dispersion is transferred to a vessel with continued mixing and heating at 75-78°C.

In a separate mixing vessel, the mineral oil and oil phase ingredients are added and mixed with heating to about 80-82°C to form a uniform oil phase. The heated oil phase is slowly added to the heated water phase using high speed mechanical dispersing means and mixing is continued until a homogeneous o/w emulsion is obtained through cooling to room temperature.

The following results are obtained in a comparison of moisturizing activity.

| | % Increase in Skin Softness (in vivo) | |
|---|---|---|
| Time | o/w Emulsion of Ex. IV | o/w Emulsion of Ex. IV with 0.4% w/w SANWET IM-1000 |
| 0 (baseline) | 0 | 0 |
| 2 mins. | 48.2% | 46.3% |
| 5 " | 33.5 | 45.8 |
| 10 " | 30.1 | 33.3 |
| 1 hour(s) | 21.9 | 28.4 |
| 2 " | 20.5 | 27.4 |
| 3 " | 19.5 | 27.4 |
| 4 " | 15.8 | 23.8 |
| 5 " | 16.7 | 23.3 |
| 6 " | 13.5 | 23.8 |
| S.I. | 1.0 | 1.32 |

Example VI

In this example, the following multiphase (double emulsion) oil-in-water-in-silicone fluid skin care emulsion in lotion form is prepared from the following ingredients (indicated by chemical or CTFA name).

| Ingredient | % w/w |
|---|---|
| Aqueous Phase: | |
| Purified water | 61.17 |
| Pantethine, 80% aqueous solution (humectant) | 0.10 |
| Methylparaben (preservative) | 0.20 |
| Carbomer 940 (thickener) | 0.10 |
| Glycerin (humectant) | 2.50 |
| Sodium alkyl polyether sulfonate (anionic emulsifier) | 1.25 |

| Ingredient | % w/w |
|---|---|
| Oil Phase: | |
| Heavy mineral oil | 1.75 |
| Cholesterol (emollient & auxiliary emulsifier) | 1.00 |
| Cetyl palmitate (auxiliary emulsifier) | 0.20 |
| PEG-22/Dodecyl glycol copolymer (emulsifier) | 0.20 |
| Ethylparaben (preservative) | 0.10 |
| Propylparaben (preservative) | 0.15 |
| Neutralizer Base: | |
| Triethanolamine | 0.10 |
| Color and Frgrance: | |
| FD&C Red No. 4 (1% aqueous solution) | 0.03 |
| Odorant oil | 0.30 |
| Silicone Phase: | |
| Cyclomethicone/Dimethicone copolyol (90:10) | 9.50 |
| Clyclomethicone/Mimethiconol (13:87) | 5.00 |
| Cyclomethicone | 3.00 |
| Phenyl Dimethicone | 1.00 |
| Pareth-15-3 (auxiliary emulsifier) | 2.00 |
| Octyl Methoxycinnamate (U.V.-absorber) | 7.00 |
| Benzophenone-3 (U.V.-absorber) | 0.50 |
| $C_{12-15}$ Alcohols Benzoate (solubilizer) | 2.85 |
| | 100.00 |

The aqueous phase ingredients other than the Carbomer 940 thickener are heated and mixed together followed by addition of the Carbomer 940 as in Example 1 and maintained at about 72-75°C with constant agitation. The oil phase ingredients are mixed together in a separate vessel and maintained at about 80-85°C. The heated oil phase is then slowly added to the heated water phase, as in Example 1, to form the o/w emulsion (about 70-75°C). After neutralization of the Carbomer 940 by addition of the triethanolamine (about 60°C), the emulsion is further cooled with continued moderate mixing and the colorant (about 45-50°C) and fragrant oil (about 35-40°C) are added, followed by cooling to room temperature (about 25°C).

The four silicone fluids and other silicone phase ingredients are mixed together in a separate vessel until a uniform phase is attained. The o/w emulsion is slowly added to the silicone phase with stirring until a homogeneous o/w/s double emulsion in lotion form is attained.

Example VII

The oil-in-water-in-silicone fluid (o/w/s) emulsion of Example VI is modified by incorporating 0.1 weight percent of SANWET IM-1000 (FG) into the aqueous phase after the Carbomer addition, using high shear mixing until a uniform dispersion is obtained. The following results are obtained in a comparison of moisturizing activity.

| Time | % Increase in Skin Softness (in vivo) | |
|---|---|---|
| | o/w/s Emulsion of Ex. VI | o/w/s Emulsion of Ex. VI with 0.1% w/w SANWET IM-1000 |
| 0 (baseline) | 0 | 0 |
| 2 mins. | 45.3% | 52.0% |
| 5 " | 40.6 | 41.5 |
| 10 " | 29.8 | 36.5 |
| 1 hour(s) | 23.4 | 24.4 |
| 2 " | 23.6 | 22.3 |
| 3 " | 15.4 | 15.4 |
| 4 " | 13.7 | 14.4 |
| 5 " | 7.8 | 10.3 |
| 6 " | 6.0 | 12.2 |
| S.I. | 1.0 | 1.14 |

Example VIII

In this example, the indicated amount of SANWET IM-1000, granular, regular grade (RG), is incorporated into the aqueous phase of a commercial skin moisturizing o/w emulsion oil (Oil of Olay® Beauty Fluid, Richardson-Vicks Inc., Wilton, CT) and tested for enhanced moisturizing activity. The following results are obtained.

| Time | % Increase in Skin Softness (in vivo) | | |
|---|---|---|---|
| | Commercial product | With 0.05% w/w SANWET IM-1000 | With 0.1% w/w SANWET IM-1000 |
| 0 (baseline) | 0 | 0 | 0 |
| 2 mins. | 58.0% | 57.1% | 58.9% |
| 5 " | 42.0 | 45.9 | 54.3 |
| 10 " | 40.0 | 46.4 | 49.0 |
| 1 hour(s) | 26.4 | 29.6 | 30.4 |
| 2 " | 23.9 | 29.6 | 32.4 |
| 3 " | 22.7 | 22.8 | 27.6 |
| 4 " | 23.2 | 22.7 | 24.4 |
| 5 " | 18.8 | 16.2 | 21.1 |
| 6 " | 18.7 | 17.0 | 20.4 |
| S.I. | 1.0 | 1.11 | 1.16 |

The emulsion composition of this example, when rubbed on the face in the usual manner for applying a facial

lotion, provides a non-oily coating that moisturizes and softens the skin.

Example IX

This example demonstrates the increased moisturizing activity obtained by incorporation of a smaller particle sized starch-based water-absorbable resin composition into the aqueous emulsion phase. The following results are obtained in a comparison of SANWET IM-1000 fine grind (FG) with SANWET IM-1000 regular grind (RG) in the same commercial product of the preceding example.

| | % Increase in Skin Softness (in vitro) | |
|---|---|---|
| Time | With 0.1% w/w SANWET IM-1000 (RG) | With 0.1% w/w SANWET IM-1000 (FG) |
| 0 (baseline) | 0 | 0 |
| 2 mins. | 68.7% | 67.2% |
| 5 " | 54.3 | 67.2 |
| 10 " | 37.6 | 49.9 |
| 1 hour(s) | 25.1 | 37.4 |
| 2 " | 16.7 | 29.4 |
| S.I. | 1.0 | 1.38 |

Analysis of SANWET IM-1000 (RG) indicates a particulate mesh size (standard U.S. Serial Sieves) of not more than about 1% greater than 100 mesh; at least about 84% between 20-145 mesh; and not more than about 15% less than 145 mesh. Analysis of SANWET IM-1000 (FG) indicates a particulate size of not more than about 7-10% greater than 200 mesh; about 35% between 200-325 mesh; and about 55-58% less than 325 mesh.

Example X

In this example, the smaller particle sized SANWET IM-1000 (FG) and SANWET IM-1500 (RG) are compared, with 0.1% weight percent of each, respectively, being incorporated into the same commercial product of Example VIII. As in Example IX, the smaller particle size affords increased moisturizing action to the final product.

| Time | % Increase in Skin Softness (in vivo) With 0.1% w/w SANWET IM-1500 (RG) | With 0.1% w/w SANWET IM-1000 (FG) |
|---|---|---|
| 0 (baseline) | 0 | 0 |
| 2 mins. | 40.4% | 55.6% |
| 5 " | 45.1 | 50.6 |
| 1 hour(s) | 30.1 | 34.9 |
| 2 " | 25.3 | 30.1 |
| 3 " | 20.5 | 25.3 |
| 4 " | 20.5 | 25.3 |
| 5 " | 10.2 | 20.5 |
| 6 " | 10.2 | 20.5 |
| S.I. | 1.0 | 1.22 |

Example XI

This example illustrates a w/o/w emulsion system modified by the addition of a starch grafted sodium polyacrylate. The preparation of the emulsion is described in Example 7 of U.S. Patent No. 4,254,105, incorporated herein by reference, as follows:

Beginning of Quote

Phase (1): Oil phase made by mixing 85 g of liquid paraffin and 85 g of olive oil, heating the mixture to 70°C and dissolving 10 g of sorbitan mono-palmitate and 10 g of sorbitan monostearate therein.

Phase (2): Internal water phase made by dissolving 50 g of glucose in 300 ml of distilled water.

Phase (3): External water phase made by dissolving 6 g of sucrose fatty acid ester featuring HLB 11, and 3.0 g of polyoxyethylene sorbitan monostearate in 100 ml of distilled water.

Subsequently, (2) was added to (1) at 70°C, and the mixture was subjected to agitation by a homomixer for emulsification to prepare w/o emulsion.

250 g of the w/o emulsion was added to (3) preheated to 70°C, and the mixture was gently agitated by a homomixer, with consequent w/o/w emulsion.

End of Quote

For purposes of this example, 0.01 weight percent of SANWET IM-1000 (FG) is added to each of Phases (2) and (3) with homomixer dispersion and the thus-modified w/o/w lotion emulsion is prepared as described above. In comparison with the w/o/w emulsion with no resin gellant, enhanced skin moisturization and larger S.I. value is obtained.

**Claims**

1. A skin moisturizing emulsion composition comprising at least one aqueous phase having uniformly dispersed therein an effective skin moisturizing amount of a water-absorbent resin polymerizate obtainable by a process which comprises polymerizing (A) at least one polysaccharide selected from the class consisting of starches and cellulose, (B) at least one monomer having a polymerizable double bond which is water-soluble or becomes water-soluble by hydrolysis, and (C) a cross-linking agent, and if the monomer (B) is one which becomes water-soluble by hydrolysis, thereafter subjecting the resulting product to hydrolysis.

2. The emulsion composition of Claim 1 wherein said polysaccharide is a monomer selected from the group consisting of natural starch and alpha starch.

3. The emulsion composition of Claim 1 wherein said monomer which is water-soluble is a monoethylenically unsaturated compound selected from the group consisting of (meth)acrylic acid,

anhydride thereof or salt thereof.

4. The emulsion composition of Claim 3 wherein said salt is the sodium salt.

5. The emulsion composition of Claim 3 wherein said cross-linking agent is a bis-(meth)acrylamide.

6. A skin moisturizing emulsion composition comprising at least one aqueous phase having uniformly dispersed therein an effective skin moisturizing amount of a starch-based water-absorbent resin polymerizate obtainable by a process which comprises polymerizing a natural starch, a water-soluble monoethylenically unsaturated monomer having a polymerizable double bond and selected from the group consisting of (meth)acrylic acid, anhydride thereof or salt thereof, and the cross-linking agent, N,N-alkylene($C_1$-$C_6$)bis(meth)acrylamide.

7. The emulsion composition of Claim 6 wherein said salt is the sodium salt.

8. The emulsion composition of Claim 6 wherein said acrylamide is N,N-methylene-bisacrylamide.

9. The emulsion composition of Claim 6 wherein said aqueous phase contains at least about 0.01 weight percent of said starch-based water-absorbent resin polymerizate.

10. The emulsion composition of Claim 6 wherein said aqueous emulsion phase contains from about 0.1 to about 5 weight percent of said starch-based water-absorbent resin polymerizate.

11. The emulsion composition of Claim 6 which also contains an effective sunscreening amount of a cosmetically acceptable sunscreening agent.

12. The emulsion composition of Claim 6 which is an oil-in-water emulsion.